# EUROPEAN PATENT APPLICATION

(11) **EP 2 156 844 A1**
(43) Date of publication of application: **24.02.2010**
(21) Application number: 08767027.9
(22) Date of filing: 29.04.2008
(51) Int. Cl.: A61K 39/04, C12P 21/00, C12N 1/21, G01N 33/531

(54) **AGENT EXHIBITING PROPERTIES TO FORM THE CELLULAR IMMUNITY AGAINST MYCOBACTERIUM TUBERCULOSIS H37 Rv, METHOD FOR THE PRODUCTION THEREOF (VARIANTS), A RECOMBINANT STRAIN AND AN AGENT FOR TUBERCULOSIS DIAGNOSIS**

(30) Priority: 10.05.2007 RU 2007117342
(71) Applicant: Obschestvo S Ogranichennoy Otvestvennostyu "BIOTEK", Moscow 127238 (RU)
(72) Inventor: KISLICHKIN, Nikolay Nikolaevich, Moskovskaya obl., 142279 (RU)
(74) Representative: Maiwald Patentanwalts GmbH
(86) International application number: PCT/RU2008/000270
(87) International publication number: WO 2008/140353

(57) **Abstract**

The invention relates to biopharmacology, preparative biochemistry and medicine, in particular to an agent which is based on a tuberculous complex produced by a microorganism and which can be used for tuberculosis prevention, treatment and diagnosis. The recombinant 2-9XL Acinetobacter johnsonii strain, which is harmless for man, animal and environment, comprises, in the composition of the chromosome thereof, the fragment(s) of a chromosomal DNA M. tuberculosis H37 Rv, which makes it possible to simultaneously synthesise several proteins for forming a stable species-specific tuberculous glycoprotein complex in the outer membrane and capsularsubstance of a bacterium. Ease of use of the recombinant 2-9XL strain, the use of standard media, the stability of the species-specific tuberculous complex synthesis, the cost-effective and safe practice for releasing and purifying the tuberculous complex render said microorganism a potential producer of the agent exhibiting properties to form the T- and B-cellular immunity against an M. tuberculosis originator.

## Description

The invention is referred to the a bio pharmacology branch, a preparation bio chemistry, medicine and concerns development of a tool, based on the tuberculosis complex, produced by a microorganism, which can be applied in treatment and diagnostics of tuberculosis.

An important part in solving the tuberculosis problem is production of biological vaccines and development of fast and inexpensive diagnostic methods. It is clear, that required is to identify in the tuberculosis agent such proteins and complexes they form, which are to meet the latest demands to achieve these purposes. The principle criterion of biological vaccines are their safety for people and high efficiency, the ones of diagnostic preparations are susceptibility and specifics. Major directions of the stated problem solution are as follows: a) gene cloning of mycobacteria in a safe recipient and studying properties of the produced tuberculosis proteins, b) producing from mycobacteria proteins and their complexes with the following properties specification.

In literature described is the facility to form genome library M. tuberculosis H37 Ra and transferring fragments of chromosome DNA of a tuberculosis agent in recipient bacteria E. coli XL1-Blue. It is shown, that from a certain fragment of chromosome DNA M. tuberculosis H37 Ra, which consists of 1535 base pairs and is in the structure of pZx7 plasmid vector, and possible is synthesis of a tuberculosis protein with an approximate mass 52, 0 KDA. This protein is located on the outer membrane E. coli XL1-Blue, makes the recombinant strain to penetrate into eukaryotic cells HeLa and proliferate in them, which is typical for pathogenic mycobacteria. And yet the authors do not point out that the given protein is individual (type-specific) and might be used to diagnose the tuberculosis agent. Contrariwise, an amino acid sequence of the examined protein has a homo-geology both with pathogenic microorganisms (Listeria monocytosis, Shigella, Jersinia pseudotuberculosis), and a set of human proteins, for instance, p- adaptyne[1].

From filters of M. tuberculosis culture produced and characterized is a released protein MPT63, with a molecular mass 18, 0 KDA. Analysis of a nucleus sequence tr+63 gene has shown that it has an open reading frame, coding a protein from 159 amino acids and consisting of 29 amino acids of a signal peptide and 130 amino acids of a mature MPT63 proteins. The recombinant MPT63 protein from cells E. coli and refined from cultural filters M. Tuberculosis were invisible in serologic reactions and affected greatly on an anti-body immunity of guinea pigs, infected by a virulent strain M. tuberculosis. It was supposed to use this protein as a specific reagent to diagnose a dermal testing under tuberculosis, however, till now this protein and a protein complex with a signal peptide have not been used widely [2].

Also known are description and specification of 4 main extracellular proteins M. tuberculosis, produced from non-pathogenic rapidly developed mycobacteria, which have a ferment activity and are found also in pathogenic strains. These proteins are to be used for constructing biological vaccines, as they induce a protective immunity, nevertheless, till now they are unrecognized [3].

Therefore, up to now there is no any gene - engineer microorganism, which could synthesize tuberculosis type-specific proteins and their complexes, suitable to be used for diagnostics and to be a basis to develop a biological vaccine. Proteins, produced from pathogenic strain M. tuberculosis or non-pathogenic Mycobacterium, till now do not make possible to produce on their basis highly specific and susceptible diagnostic preparations or construct biological vaccines.

The invention is objected to produce by a genetic - engineer and microbiological method a recombinant strain 2-9XL Acinetobacter johnsonii - a producer of a specific tuberculosis M. tuberculosis H37 Rv, which can be a basis of the biological vaccine against the tuberculosis agent and the diagnosticum to reveal people suffering tuberculosis with various forms of the disease at its different stages.

To solve the set problem offered is a recombinant strain 2-9XL Acinetobacter johnsonii, produced by a genetic - engineering method with the next selection by a microbiological method, being a producer of a type-specific glycoprotein tuberculosis complex (TB - antigen), which is located on the outer membrane and in a capsule bacteria substance and at producing can be used as a basis to develop a biological vaccine, diagnostic and drug preparations.

The technical result is producing highly specific and susceptible preparations.

To solve the set problem offered is a group of inventions, united under the general inventory idea.

Declared is the invention, being a preparation with a property to form a cellular immunity against M. tuberculosis H37 Rv. The preparation contains a type-specific glycoprotein tuberculosis complex M. tuberculosis H37 Rv (TB - antigen).

Besides that, declared are variants to produce a type-specific glycoprotein tuberculosis complex M. tuberculosis H37 Rv (TB - antigen), as well as a recombinant strain 2-9XL Acinetobacter johnsonii VKPM-9312 - the producer of a type-specific glycoprotein tuberculosis complex M. tuberculosis H37 Rv (TB-antigen) and the tool to diagnose tuberculosis, containing the above-stated type-specific glycoprotein tuberculosis complex.

2-9XL Acinetobacter johnsonii is a genetic - engineering strain, produced by transferring into bacteria of R-form F. tularensis in structure of the vector plasmid pRT the two fragments of a chromosome DNA M. tuberculosis H37 Rv (Annexes 1 and 2) and the next selection by a microbiological method.

Up to now at gene cloning of the tuberculosis agent (TB) in structure of vector plasmids used have been bacteria - proprietors, which could synthesize only particular proteins TB. Production and analysis of TB genetic - engineering proteins (as well as natural proteins of tuberculosis agents) have not resulted in what was desired - development of biological vaccines, medicinal preparations, highly specific and susceptible diagnosticums. Therefore, the world goes on searching new mycobacteria proteins, by means of which it is possible to solve the set problems.

Potentially, the given direction might be erroneous, as a type-specifics of the tuberculosis agent is formed at level of the complex from several proteins (no less than three), a part of them should be glycolysized that is to contain a polysaccharide component.

It is shown that at transferring M. tuberculosis H37 Rv DNA fragments in structure of the vector plasmid pRT into the recipient R-from F. tularensis and the next selection of a trans formant by a certain microbiological method, the transferred fragments (fragment) are inbuilt in a chromosome DNA of the bacteria - recipient and result in synthesizing the tuberculosis polypeptide. Primarily this polypeptide forms in the bacteria - proprietor a specific tularemia - tuberculosis complex by connecting with tularemia proteins with a higher molecular mass. However the given construction is likely to be inconvenient for the bacteria - proprietor and at further selecting a recombinant strain started is the synthesis of TB - proteins with a higher molecular mass, which substitute the tularemia ones.

At such a serious "reconstruction" in a bacterium changed are not only biochemical and pheno-typical properties of the recombinant microorganism (by a set of properties it becomes like tuberculosis mycobacteria), but changed is also the structure of a DNA - bacterium.

The type belonging conclusion of "Ekha" strain (2-9XL) by the analysis 16S RNA is made in the Research Institute for Genetics and Selection of Industrial Microorganisms.

The sequence analysis 16S pRNA has shown that the examine strain, declared as "Ekha" (2-9XL), is referred to the type of Acinetobacter johnsonii bacteria, at that with the strain Acinetobacter johnsonii strain 42 the homo- geology makes 99%.

Primary properties of the strain 2-9XL Acinetobacter johnsonii - a super producer of the recombinant tuberculosis antigen.

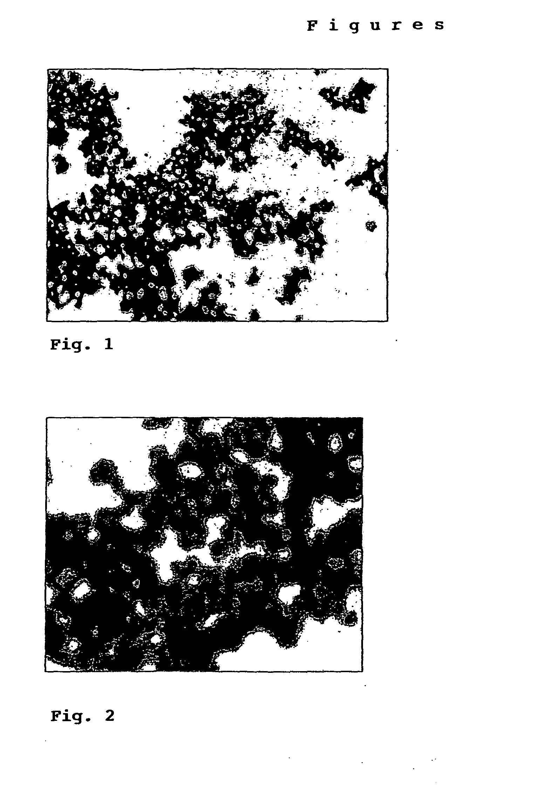

Cultural - morphologic properties: At optical microscopy (Fig.1. A-B. Optical microscopy of bacteria 2-9XL Acinetobacter johnsonii. Staining reaction under Gram (Set of colors by "Serva" firm). The culture 2-9XL Acinetobacter johnsonii is grown on the tularemia medium (FT-agar with 1% glucose and additives, pH 6.6) within three days at +32°C. A-UV 100HZ...; V-100H9, HZ... (Microscope "Biomed-2", Russia). On the presented figures it is clearly seen, that the recombinant bacteria 2-9XL are stable to de-coloring and tended to aggregation (adhesion). The bacteria capsule is colored by safranin into red; the bacteria are immovable polymorphic small coccoid and bacillus cells with sizes of 0.4-1.0 MKM. Under Gram coloring bacteria are resistant to de-coloring, which are appeared Gram positive colored. They form agglomerations of cells different by sizes and forms, which indicates a high level of their aggregation (adhesion). The bacteria aggregation is connected with forming a specific capsule, which is well-colored by safranin. At losing the recombinant tuberculosis antigen, bacteria become gram - negative.

The strain 2-9XL aerobe is developed on simple and saturated mediums (auxotroph) at +32°C, though it can grow in the interval from +28°C to +37°C. At bacteria growing on simple mediums the culture stops synthesizing the recombinant tuberculosis antigen. The require growth factor for 2-9XL is cysteine. For maximum synthesis of a specific capsule, containing the recombinant tuberculosis antigen, preferable are saturated mediums with vitamin and other additives.

As a stimulator of the bacteria growth and forming a specific capsule it is possible to add into a culture media 1% suspension of autoclaved bacteria 2-9XL and (or) 10% autoclaved capsule substance. The most suitable for growing the recombinant strain 2-9XL are culture medium, used to grow tularemia, plague, brucellosis, etc. The bacteria inoculations on the culture media are kept in thermostat from two up to seven days. At growing the bacteria 2-9XL there is a specific, typical, non-irritating smell. 2-9XL strain does not grow well on liquid culture media and quickly lacks its ability to produce the recombinant tuberculosis antigen.

On solid culture media 2-9XL bacteria form colonies of a yellowish grey-white color. At titrating the culture to separate isolated colonies possible is the growth delay up to two days. As they are growing the colonies can have the diameter of 5-7 mm. If the inoculation plates are kept 2-3 days in the fridge (+4°C), the colonies become mainly of yellow-gray color. The colonies are raised with unsmooth edges, unpolished, non-transparent, when looping the colonies are of solid consistency, easily removed from the culture agar surface. At titrating 2- 9XL culture on solid culture media it is impossible to prepare a standard suspension due to a high ability of the cells to adhere (spontaneous agglutination), therefore the bacteria titer does not correspond to the standard raising (Fig. 2. A-C. Cultural - morphological properties of 2-9XL Acinetobacter johnsonii. Isolated colonies of the recombinant strain 2-9XL Acinetobacter johnsonii, grown on FT-agar with cysteine and 1% glucose, pH 6.6 within three days at +32°C. To be kept in the fridge (4°C) within five days. A - common background; B - C - white background. C - is an enlarged fragment of Petri dish.

The population stability of 2-9XL strain.

At sequential sub-culturing on solid culture media, growing at various temperature regimes, after keeping in a lyophilic dried condition, at worsening the medium nutritious properties for rising and other unfavorable conditions the strain 2-9XL becomes instable and is dissociated in other forms. Dissociation is more remarkable after a long-term rising of isolated colonies (Fig. A-C. Dissociation of the culture 2-9XL Acinetobacter johnsonii on cultural - morphological signs).

Petri dish with a dissociated culture 2-9XL. Growth of colonies with various morphology at the culture titration from a lyophilic dried condition, FT-agar with 1% glucose, cysteine, pH 6.6, time of incubation at +32°C - 10 days, kept in the fridge (+4°C).
A - Petri dish is photographed on a white background.
B - Petri dish is taken on a grey background
C (1-5) - Enhanced fragments of Petri dish with colonies 2-9XL of various morphology.
C (1) - A large, round, white, non-transparent, flat, with a raised center and smooth edge, glossy colony, easy removable from the agar by looping;
C (2) - A small, round, grey-yellow, non-transparent, raised, with a smooth edge, unpolished colony, easy removable from the agar by looping
C (3) - A mid-sized, round, grey-yellow-white non-transparent with a raised well visible center and an expressed roller in the smooth edge, unpolished colony, easy removable from the agar;
C (4) - A mid-sized, non-round, yellow, non-transparent, with a well visible raised center, unsmooth surface and edge, unpolished colony, easy removable from the agar by looping
C (5) - A large, non-round, yellow-grey, non-transparent, with a visible raised center, uneven surface and edge, unpolished colony, easy removable from the agar by looping). Biochemical properties of 2-9XL strain. Bacteria under growing on solid culture media split proteins with emission of a specific, non-irritating smell. At growing on saturated nutritious media are capable to synthesize the recombinant tuberculosis antigen. Oxidase-positive and catalase- negative.

Biochemical properties of the recombinant strain 2-9XL Acinetobacter johnsonii are not studied in full.

Immunological properties. 2-9XL bacteria are of a very high adhesiveness (ability to agglutinate). (See Fig1. A - B). And it does not make possible to check an agglutination ability of them in an agglutinating reaction.

In reactions of immune-diffusion (RID) and immune - electrophoresis with experimental serums, produced on ultrasonic mycobacterium disintegrators of the virulent tuberculosis strain M. tuberculosis H37 Rv, antigens of recombinant bacteria 2-9XL do not form precipitation lines. It is probably, due to antigens to the recombinant TB - antigen appearing only in process of the infection development of a human body or animals and they do not have a large titer. In case of raising Mycobacterium on culture media and their nest inactivation by acetone (or other hard bactericidal preparations), this antigen is destroyed, and therefore no specific anti-bodies can't be produced.

In the immune ferment analysis (IFA) TB - antigens in bacteria ultrasonic disintegrates and in a refined form interact with the blood serum anti-bodies of people suffered with tuberculosis and do not interact with anti-bodies in blood serums of healthy people. In an immune blotting TB - antigen is not to interact with anti-bodies of people suffered tuberculosis, as it is not transferred to nitro-cellulose membrane.

Relation to phages of the given type. For the recombinant strain 2-9XL Acinetobacter johnsonii till now no proper phages are detected.

Genetic features. The strain 2-9XL Acinetobacter johnsonii in structure of the chromosome DNA contains as minimum one fragment inset of the chromosome DNA M. tuberculosis H37 Rv, but possible are also two insets (Annexes 1 and 2).

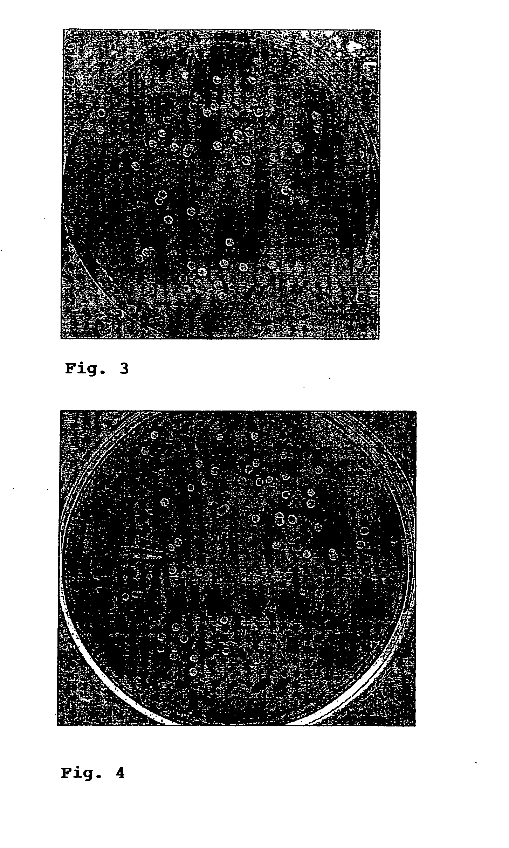

Productivity. The recombinant strain 2-9XL is a producer of the tuberculosis antigen (TB - antigen) of a glycoprotein nature. Proteins, synthesized from a fragment of the chromosome DNA M. tuberculosis H37 Rv (See Annex 1), form on the outer membrane bacteria 2-9XL and in its capsule substance a type-specific glycoprotein complex (Fig. 4). At producing ultrasonic disintegrates of bacteria (USD) this complex is parted and is available in the form of several proteins. At concentrating USD by trichloroacetic acid, proteins of TB - antigen are collected into a specific recombinant tuberculosis complex. At a sparing producing the recombinant TB-antigen from bacteria 2-9XL its molecular mass in the denatured SDS-PAAG is in the interval from 55.0 to 75.0 KDA (Fig.4).

To the described glycoprotein complex lab animals have a formed continuous and tensed B and T immunity against M. tuberculosis H37 Rv. At studying an anti-body production (formation of B- cellular immunity) to the recombinant TB - antigen immunized were guinea pigs and rabbits in order to produce hyper-immune serums. For immunization used were rabbits with weight of 2.5-3 kg. 1 ml TB-antigen in concentration of 1 mg, mixed with an equal volume of Freind incomplete adjuvant was injected hypodermic to the back and shovel area. The immunization was repeated three times with a week interval and double dose increase. After the first immunization cycle was over, in 4 weeks there was a repeated intramuscular injection of preparations, starting from 2 mg and multiply increasing the dose to 8 mg. Guinea pigs were immunized hypodermic to the back and shovel area by TB - antigen in dose of 300 mcg/ml with Freind incomplete adjuvant. In three weeks the preparation was injected repeatedly.

An anti-body reaction of the lab animals was valued in 21 days after the last injection of TB - antigen. For that taken was the blood, produced was the serum and tested were available specific TB - antigens in restriction of the diffuse precipitation and by an immune ferment method. It was shown that specific antibodies are generated already after the first preparation injection. At further injections, antibodies 'titers in serums were increased. After immunization cycles being over, produced were hyper immune serums with a high content of TB - antigens. In an immune ferment analysis with using TB - antigen, taken for immunization, antibodies titers of guinea pigs made 1: 6400, of rabbits -1: 409600 and more, in reaction of the diffuse precipitation the titers made 1:8 and 1:16-1:32 accordingly.

Therefore, an available recombinant TB - antigen in animals' body results in reconstructing their immune system under B -type. A continuous available TB - antigen or its repeated injection makes a stable reconstruction of the immune system, which is proved by the enlarged titers of specific antibodies in blood serums of animals.

Tubercular people and bacteria carriers have a specific B- cellular immunity which is defined by TB - antigen in immune ferment test - systems (IFA), on nitrocellulose filters (Immune blotting, Dot-blot) and in other reactions with using clinic liquids (blood serum, phlegm, pleural fluid, back- cerebral liquid, etc), containing specific antibodies.

Testing blood serum samples of tubercular people and healthy people to available specific antibodies is given in the table.

Testing a specific activity of TB - antigen in formation of T - cellular immunity was made by tubercular sampling guinea pigs. For that groups of guinea pigs were injected by tuberculosis vaccine BCG (intra-dermal of 0.5 mg in 0.2 ml diluted fluid) and suspension of microbe cells of virulent strains Mycobacterium tuberculosis H37R_{V}, R1R_{V}, as well as atypical strains M. kansassi, M. marinum (intra-peritoneal in a dose of 10⁶ microbe cells per an animal). In 4 weeks after injecting, the observed and tested guinea pigs were used.

To sample a Mantoux test under the general procedure / INSTRUCTION on using tuberculosis refined dried allergen for a dermal, hypodermic and intra-dermal application (dried refined tuberculin). Chief administration of a medical-prophylactic aid under Ministry of Health USSR. Moscow. 11 August 1986/ Concentration of 5 preparations, dermal injected, made 10 mcg on the general protein in 100 mcl diluted fluid. A specific allergic reaction of the delayed type in form of a local reaction like a cellular infiltration (papule) was accounted in millimeter in 24 hours. A preparation to compare was a dried refined tuberculin (Tuberculinum depuratum siccum) produced by RAO "BIOPREPARAT", the Saint-Petersburg Research Institute of Vaccines and Serums - PPD-L-2 (an intra-dermal injection dose is - 25 TE).

Conditions and structures of media for the strain storage and maintenance. A museum culture of the strain 2-9XL Acinetobacter is kept at temperature +4°C on stocks of FT-media with additives of glucose and cysteine up to 2 months, and in a lyophilic dried condition in the sucrose - gelatin media up to 5 years.

Fermentation medium. The recombinant strain 2-9XL Acinetobacter johnsonii is fermented on a solid FT- medium with additives of glucose and cysteine at +32°C.

Technological peculiarities at fermentation. The recombinant strain 2-9XL grows badly in liquid culture media and quickly lacks an ability to produce TB - antigen. Therefore the biomass increase for further development of TB- antigen is made only on solid growth media.

The matrix culture is grown on slopes at +32°C within 2-3 days, then bacteria are washed off by a physiological solution and from one slope seeded are one-two mattresses. Mattresses are incubated in a thermostat at +32°C within 3-4 days, and then the raised biological mass is washed off by distillated water and by extraction released from soluble components of the growth medium. From the biological mass, cleared from the growth medium components started is producing the recombinant TB - antigen.

The invention is illustrated by the next examples.

### Example 1. Producing a type-specific glycoprotein tubercular complex (TB - antigen) from the recombinant strain 2-9XL Acinetobacter johnsonii.

On the solid growth medium (slopes) pH 6.6, containing from a cardio-cerebral agar, hydrolyzed hemoglobin, cysteine and glucose in corresponding proportions or FT-agar pH 6.6 (Produced by FSUE SSC AM, Obolensk, Russia) with addition of cysteine and glucose by looping seeded is the culture 2-9XL. The seeding is incubated in a thermostat at +32°C within two days. A two-day culture is washed off by a physiological solution and prepared is the bacteria suspension in concentration of 5x10⁹ microbe cells (mc) per a milliliter. [It is hard to prepare a suspension of 2-9XL bacteria, as the culture is washed off in flakes, which are difficulty split at mixing]. To prepare a lot of biological mass, the made suspension is seeded on mattresses at one slope per 1-2 mattresses (Into one mattress poured is 400.0 ml growth medium). The seeding is incubated in a thermostat at temperature +32°C within three days. From one mattress a three-day culture 2-9XL is washed off by 30 ml physiological solution.

From the washed off culture suspension prepared is a smear for a light microscopy and it is colored under Gram. [A bacteria population of the recombinant strain 2-9XL under slight changes in the growth medium structure, temperature or other conditions can quickly dissociated in forms, which are not to produce TB - antigen. Under Gram coloring these bacteria are gram - negative, bacteria - producers of TB - antigen are resistant to de-coloration and look like gram - positive (See Fig.1. A - B). In the raised bio mass 2-9XL an amount of gram - negative bacteria is not to exceed 5-10%]. A suspension of the bacteria washed off from the mattress is extracted ("Beckman" G2-21, USA) at 9000 turn/ min within 30 minutes. A supernatant fluid, containing soluble components of the growth media are rejected, and the residue is suspended in 40 ml of de-ionized water and all the next procedures are carried out at +4°C. Further on there is a treatment of 2-9XL 0.5N culture by NaOH solution, increasing pH from 6, 0 to 9, 0. At that the bacteria capsule containing a type-specific recombinant TB-antigen is dissolved separately, by little portions and carefully mixing suspension within several hours. The bacteria suspension processed in such a way is left in the fridge (+4°C) for a night (15-16 hours).

The next day the bacteria suspension 2-9XL is centrifuged at 12000 turn/ min within 40 minutes. The water fraction, containing a dissolved capsule of the recombinant strain 2-9XL, is separated from the residue. Due to the capsule incomplete dissolving, a procedure of the alkaline processing the residue is repeated in the same sequence.

A first extracted portion of the capsule preparation 2-9XL is coldly neutralized by 0,5 N solution of a trichloroacetic acid (TCA) up to pH 6.0 and, as it contains a lot of bacteria, it is centrifuged at 15000 turn/ min within 40 minutes. The residue is rejected and a supernatant fluid is acidulated by 0,5 N TCA to pH 3.0-3.5. At that TB - antigen is collected in a complex and starts precipitating. The preparation is left in the fridge (+4°C) for a night (15-16 hours). TCA - residue, laid-down for the night in this solution might be kept at +4°C up to a fortnight, not losing the specific activity of TB - antigen being in it. Overnight in the fridge left is the second portion of preparation to form TB - antigen residue.

The third day the first preparation portion with the laid-down residue is centrifuged at 9000 turn/min within 30 minutes. A supernatant is rejected. Per a mattress the residue is dissolved in 1-2 ml PBS- buffer (0.0067M KH₂PO₄, 0.0067M Na₂HP0₄, 0,138M NaCI, 0.0027M KCI pH 7,0). In PBS-buffer solution there is mainly a type-specific TB - antigen (See Fig. 4A), which is already revealing its specific activity, but needs an additional clearing.

The second preparation portion, left overnight, is to repeat all the required procedures (is to be neutralized by0, 5 N TCA to pH 6.0, released from the rest cells, acidulated by 0,5 N TCA to pH 3,0-3,5 and a solution with the residue laying-down is left overnight). TCA-residue, laid-down overnight can be stored at +4°C to a fortnight without losing a specific activity of its TB - antigen.

The next day the second portion of preparation is dissolved in 1-2 ml PBS - buffer. It contains mainly a type-specific TB - antigen.

An available TB - antigen, produced by the above - stated method, is registered in SDS-PAAG electrophoresis gel (Fig. 4A).

TB - antigens of the first and second portions are combined and subjected to further clearing. As a result of the made experiments it is stated that: a) a recombinant type-specific TB - antigen is located in outer structures of bacteria 2-9XL (an outer membrane and capsule substance) and can be extracted and concentrated by the above-stated procedures, b) at making the above-stated procedures, bacteria of a recombinant strain 2-9XL is practically not destroyed and a share of the produced TB - antigen is sufficiently high as compared with others substances in the solution, such as proteins, lipids and polysaccharides of bacteria. Hereby it may be considered that the made Example is the most economic method to produce TB - antigen from the recombinant strain 2-9XL Acinetobacter johnsonii, but it does not exclude the facility to produce it by any other method (Breakdown of bacteria by means of ultrasound or by freezing-thaw, or in any other way, with the next concentration of TB - antigen).

### Example 2. Producing a type-specific glycoprotein tubercular complex (TB - antigen) from the recombinant strain 2-9XL Acinetobacter johnsonii.

Onto the solid growth media (slopes) with pH 6.6, containing from a cardio-cerebral agar, hydrolyzed hemoglobin, cysteine and glucose in corresponding proportions or FT-agar pH 6.6 (Production of FSUE SSC PM, Obolensk, Russia) by looping seeded is the culture 2-9XL. The seeding is incubated in a thermostat at +32°C within three days.

A three-day culture is washed off by a physiological solution and prepared is the bacteria suspension in concentration of 5x 10⁹ microbe cells (mc) in a milliliter.

The produced suspension is seeded onto mattresses at one slope per 1-2 mattresses (In one mattresses poured is 400.0 ml growth medium). The seeding is incubated in a thermostat at +32°C within three days. From one mattress a three-day culture 2-9XL is washed off by 30ml physiological solution. From the washed off culture suspension prepared is a smear for the light microscopy and colored under Gram. (Testing available TB - antigens in the recombinant bacteria 2-9XL).

A suspension of the bacteria, washed off from the mattress is centrifuged ("Beckman" G2-21, USA) at 9000 turn/ min within 30 minutes. A supernatant fluid, containing soluble components of the growth media is rejected and the residue is suspended in 40 ml cooled de-ionized water and all the next procedures are made at +4°C. Further the culture 2-9XL is processed by 0, 5 N solution NaOH, making pH from 6.0 to 9.0. At that the procedure of dissolving the bacteria capsule containing a type-specific recombinant TB-antigen, is fulfilled separately, by adding NaOH in small portions and carefully mixing the suspension within several hours. The bacteria suspension processed in such a way is left in the fridge (+4°C) overnight (15-16 hours).

The second day the bacteria suspension 2-9XL is centrifuged at 12000 turn/ min within 40 minutes. A supernatant fluid (a partially dissolved capsule of the recombinant strain 2-9XL, containing TB - antigen), is differed from the residue (2-9XL bacteria, having preserved and not preserved the capsule). The residue is added by 40 ml de-ionized water and repeated is the procedure of a capsule dissolving, producing the second preparation portion.

A produced preparation of the capsule 2-9XL is neutralized by 0.5 N solution of a trichloroacetic acid (TCA) to pH 6.0, and then added is ammonium sulfate [(N₄)₂SO₄] to 30% saturation. The solution is left overnight at +4°C for the residue to precipitate. The next day the preparation is centrifuged at 12000 turn/ min within 40 minutes. The residue is rejected (mainly it contains even bacteria and their large fragments). The supernatant fluid, containing mainly TB - antigen, is supplemented by ammonium sulfate to 100% saturation. The preparation is left overnight at +4°C for precipitation.

A second preparation portion of the capsule 2-9XL is neutralized by 0, 5 N solution of trichloroacetic acid (TCA) to pH 6.0, then added is ammonium sulfate [(NH₄)₂SO₄] to 30% saturation, and it is left overnight at +4°C for precipitation.

The next day TB-antigen precipitated preparation, at 100% precipitation by ammonium sulfate is centrifuged at 12000 turn/ min within 40 minutes. A supernatant is rejected, the residue is dissolved in 2.0 ml PBS-buffer pH 7.0 (per one mattress) and dialyzed against a large volume of PBS - buffer.

Within a day PBS-buffer volumes are changed not less three times to remove completely ammonium sulfate from a dialysis sac.

A second preparation portion, containing 30% ammonium sulfate, is centrifuged at 12000 turn/ min within 40 minutes and the residue is rejected, a supernatant is saturated to 100% by ammonium sulfate and left overnight at +4°C.

The next day a preparation of TB - antigen second portion, precipitated at 100% precipitation of ammonium sulfate is centrifuged at 12000 turn / min within 40 minutes. A supernatant is rejected; the residue is dissolved in 2.0 ml PBS- buffer pH 7.0 (per a mattress) and dialyzed against PBS- buffer great volume.

Within a day PBS-buffer volumes are changed no less three times to remove completely ammonium sulfate from a dialysis sac.

An available concentrated TB - antigen in PBS - buffer after dialysis of the preparation first and second portions is controlled in SDS-PAAG electrophoresis (Fig. 4B).

TB-antigens of the first and second portions are united and purified further.

Hereby, production and concentration of a type-specific glycoprotein tubercular complex (TB-antigen) from the recombinant strain 2-9XL is possible not only by its precipitating from alkaline lysates by trichloroacetic acid to pH 3.5-4.0, as shown in Example 1. Possible is to use TB-antigen precipitation methods by means of various salts, for instance, ammonium sulfate or other methods, eluates contain a refined TB-antigen with a molecular mass from 55.0 to 75.0 KDA and available carbohydrates from 20% to 50%.

### Example 3. Clarification of a type-specific glycoprotein tubercular complex (TB-antigen), produced from the recombinant strain 2-9XL Acinetobacter johnsonii.

The further clarification of a specific tubercular antigen 2-9X is made by fractioning macromolecules by a gel-filtration under low pressure with using Sephadex G-200 as a matrix.

The combined concentrated TB-antigens in PBS-buffer produced by precipitating lysates 0.5 N TCA or 100% precipitation of ammonium sulfate (Example 2) are applied to the column with size of 25x600. As an eluate used is 50 TM solution TRIS -HCI, pH 7.5, with addition of 100 mM NaCI, an elution speed - 25 ml/ hour. The peaks are collected; protein is measured under the method described by Bradford [4]. Total carbohydrate containt is defined by a phenol method [5]. Depending on a type of precipitating TB-antigen from the dissolved capsule of a microbe cell (Examples 1 and 2), a volume of the applied sample and protein concentration, eluates contain a refined TB - antigen with a molecular mass from 55.0 to 75.0 KDA and available carbohydrates from 20% to 50%.

Specific tubercular recombinant antigens are stored at +4°C in a lyophilic dried state.

An available TB-antigen and its structure, produced by an above-stated method, are registered in SDS-PAAG electrophoresis gel (Fig. 4C).

Producing a refined TB - antigen is possible not only by fractioning macromolecules by gel - filtration with using various carriers as a matrix, but also be means of an ion-exchange chromatography.

### Example 4. Using a refined type-specific TB - antigen in the human tuberculosis diagnostics.

Under latent, chronic and acute forms of the human tubercular patients in their blood formed are antibodies, being specific for the given agent - M. tuberculosis H37 Rv, which makes possible to diagnose the disease by the stated sign.

To search specific antibodies used is a test-system, which happens to be an indirect immune ferment analysis with a phase separation, where as a solid carrier used are 
96-Для поиска специфических антител используют тест-систему, которая
представляет собой непрямой иммуноферментный анализ с разделением
фаз, где в качестве твердого носителя используют 96-cavity plates.
 A substrate for ELISA is a tubercular antigen, produced from the recombinant strain 2-9X by means of chromatographic fractioning.

Into the plate cavities introduced are 100 mcl of an antigen in concentration of 10 mcg/ ml in a phosphate buffer and they are incubated within 18 hours at the room temperature. The cellules are triply washed clean by a buffered physiological solution, pH 7.2 (BPS) with 0,05% Tween 20 and "filled" within an hour by 1% solution of a bovine serum albumin in BPS. After the triple washing clean into cavities of the 1^{st} vertical line of the plate introduced are 100 mcl of blood native serum, not containing specific antibodies in dilution of 1:200 (a negative control), and from the 3^{rd} to 12^{th} vertical lines - BPS in the same volume.

Then into each cavity of the 2^{nd} vertical line added are 200 mcl of the tested serums in dilution of 1:100 and by an eight ported automatic pipette they are tittered by a double step, from the last line removed are 100 mcl. To dilute serums used is BFS pH 7.2. The plates are incubated within 1 hour at 37°C. As a positive control used is the blood serum with a previously known high titer of specific antibodies (calibration). One of the plate cavities (the last one) is left to control a substrate, adding 100 mcl of substrate to 10 mcl of conjugate. After removing non-binding antibodies by a five-time washing the plate clean by BPS - 0,05% Tween 20 in volume of 170 mcl to immune globulins added are for the repeated binding 100 mcl of antitype IgG antibodies to immune globulins A, M, G, conjugated by horseradish peroxidase (Sigma), in a work dilution of 1:5000.

The reacting mixture is incubated within 1 hour at 37°C, whereupon the cells are five times washed clean by BPS - Tween 20. A substrate solution is prepared with adding 3 mcl of 30% H₂0₂ to 10 ml of a freshly made 0.04% chromo gene AzBTS (ApliHem) in 0,1M citrate -phosphate buffer, pH 5.0 and 100 mcl solution is added to each cavity.

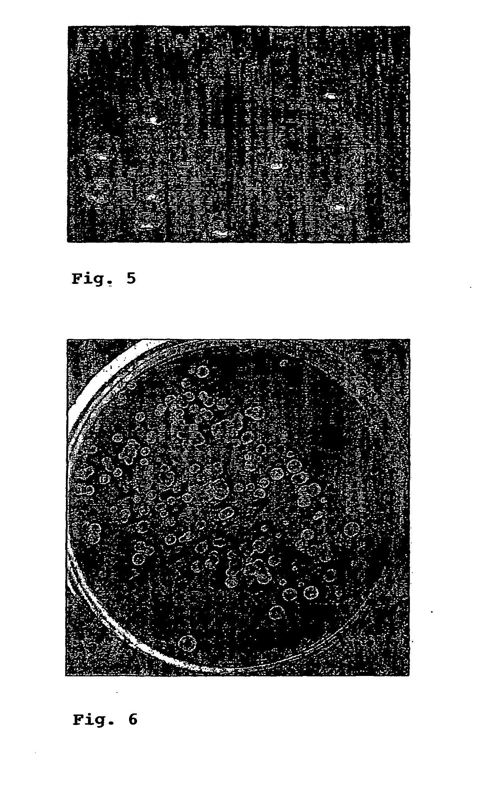

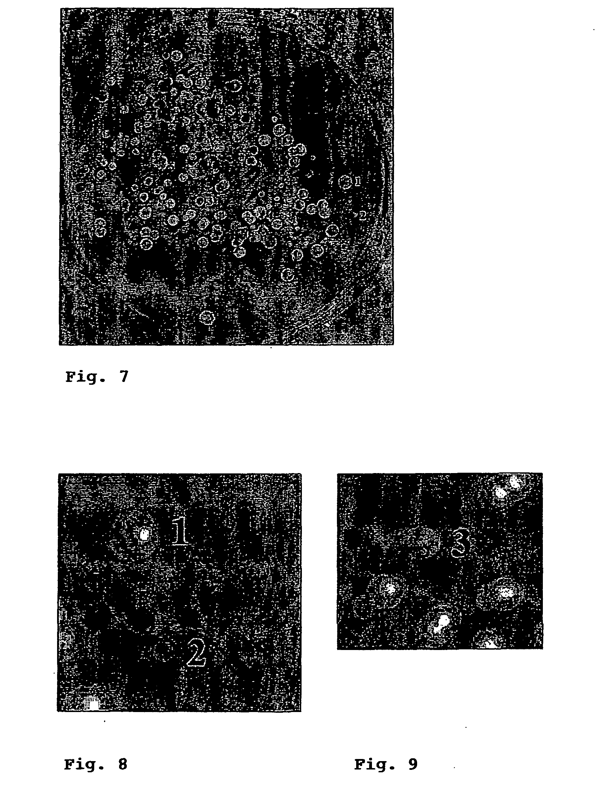

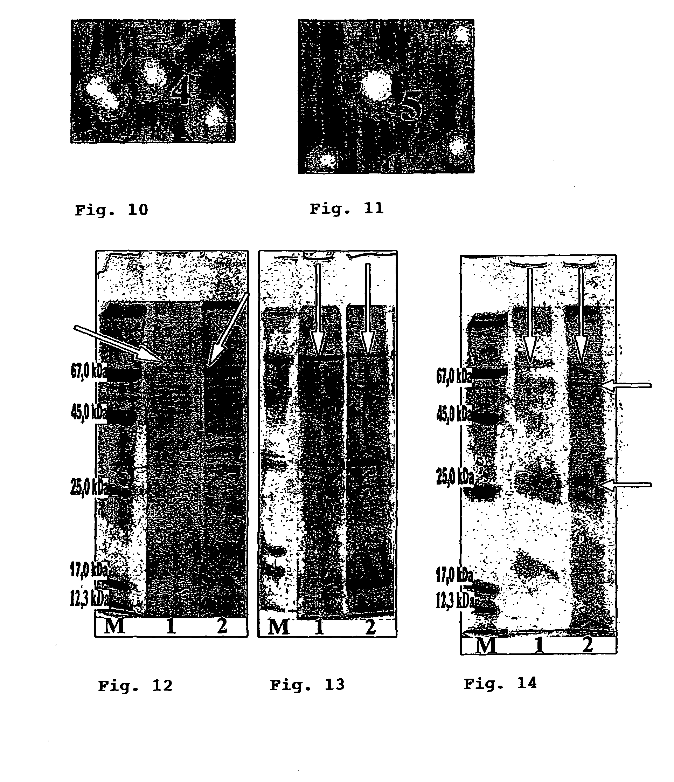

To develop a color reaction the plate is incubated at +37°C within an hour, introduce are 100 mcl of 1% SDS to stop a fermentative reaction and measured is a light absorption value at 405 nm at the vertical photometer of Bio-Rad firm, model 680. The results are compared with a blank of the vertical line cavities, in which there has been a serum without specific antibodies (Fig.5). As it is shown on Fig.5, a refined recombinant TB- antigen interacts only with blood serum antibodies of tubercular patients and does not interact with blood serum antibodies of healthy people.

Hereby, the recombinant TB - antigen 2-9XL can be used for diagnostic purposes on a solid-phase carrier, nitro-cellulose membrane and in other test-systems, by means of which identified are specified antibodies, which are produced by a human body to the available or generating tuberculosis agent - M. tuberculosis H37 Rv. Specific antibodies, produced by a human bodies in response to penetration and development in it the tuberculosis agent, can be found not only in the blood serum, but in other clinic liquids (phlegm, exudation, urine, etc).

Monoclonal antibodies or hyper immune serums of various animals (rabbits, mice, guinea pigs, etc.), produced in response to the refined recombinant TB-antigen, can be used for diagnostic methods at searching in clinical liquids of a human being (phlegm, exudation, material for micro biopsy, etc.) both tuberculosis macro bacteria and specific components.

The above-given examples of extracting and using the recombinant type-specific tuberculosis antigen, produced from a genetic - engineering strain 2-9XL Acinetobacterjohnsonii, which is safe for human beings and animals, prove, that the given microorganism is to be widely used in public health and medicine as the source of a useful product, used in diagnostics, prophylactics and treatment of the human tuberculosis.

Considering properties of the recombinant strain Acinetobacterjohnsonii 9XL, the given microorganism is deposited 29.11.05 in the All-Russian Collection of Industrial Microorganisms (ACIM) FSUE State RI Genetics under index VKPMB-9312.

List of literature:
1. S. Arruda, G.Bomfim. R. Rnights, T.Huima-Byron, L.W.Riley "Cloning of M. tuberculosis DNA Fragment Associated with Entry and Survival inside Cells". "Science", 1993, N261 (5127), p. 1454-1457.
2. C. Manca, K.Lyaschenko, H.G. Wiker, D. Usai, R. Colangeli, M.L. Gennaro. "Molecular Cloning, Purification and Serological Characterization of MPT63, a Novel Antigen Secreted by Mycobacterium tuberculosis.
3. G. Hart, Bai-yu Lee, M.A. Horwitz. "High-Level Heterologous Expression and Secretion in Rapidly Growing Nonpathogenic Mycobacteria of Four Major Mycobacterium tuberculosis Extracellular Proteins Considered to Be Leading Vaccine Candidates and Drug Targets."Infection and Immunity", June 1997, p. 2321-2328.
4. Bradford. Anal. Biochem. 72, 248 (1976), and also Anal. Biochem. 86, 142 (1978). F. Gerhard "Methods of general bacteriology" 1984, v. 2.

| Table Diagnostics of available anti-bodies to the human tuberculosis agent in blood serum samples of various men sets by an immune - ferment method (the diagnostic method is given in Example 4) | | | | |
|---|---|---|---|---|
| Blood serum samples from the diagnosed groups | Number of serum samples | Indices of optical dense (OD) | | |
| | | OD>0,6 | 0.3>O<0.6 | OD<0,3 |
| Patients from phthisiopulmonology with a phthisis diagnosis (MBT+ and MBT-) | | | | |
| | | | | |
| TB patients GISK (MBT+ and MBT-) (in %) | 227 (100%) | 162 (71,4%) | 39 (17,2%) | 26 (11,4%) |
| Donors (healthy people) | | | | |
| Donors GISK HIV, VHB, VHC (in %) | 45 100% | - - | 6 13,3% | 39 86,7% |
| Risk groups | | | | |
| Anonym donors GISK (syphilis?) (In %) | 60 100% | 4 6,7% | 9 15,0% | 47 78,3% |
| Regular soldiers, BIONOM personnel (in %) | 33 100% | 2 6,1% | 2 6,1% | 29 87,8% |
| Sportsmen GISK (in %) | 22 100% | 4 18,2% | 5 22,7% | 13 59,1% |
| Students GISK (in %) | 20 100% | 3 15% | 3 15% | 14 70% |
| Groups with possible cross-reactions | | | | |
| Patients GISK (VHV+ and VHC+) (in %) | 44 100% | 1 2,3% | 4 9,1% | 39 88,6% |
| Vaccinated GISK VHV+ (in %) | 10 100% | 1 10% | 3 30% | 6 60% |
| Note: Serum samples are provided by the State Research Institute of standardization and control over medical and biological preparations named after L.A. Tarasevich (GISK) and "BIONOM" LLC. Examined are 460 persons. OD>0.6 - indicates available antibodies in blood serums to the tubercular agent; 0,3>OD<0,6 - an uncertain result ("grey area"); OD<0,3 - indicates no antibodies to the tuberculosis agent; MBT+ - "tuberculosis" diagnosis is proved by recovering Mycobacterium from the patient; MBT- "tuberculosis" diagnosis is proved by clinical- radiologic methods. HIV - human immune deficit virus; VHV - virus of hepatitis B; VHC - virus of hepatitis C | | | | |

### Conclusions:

1. From 227 samples of tubercular patients' blood serums in 71.4% detected are antibodies to the tuberculosis agent M. tuberculosis H37 Rv.
2. In 45 blood samples of donors (healthy people) there were no antibodies to the tuberculosis agent M. tuberculosis H37 Rv. detected.
3. In risk groups in blood serum samples available antibodies to the tuberculosis agent M. tuberculosis H37 Rv make from 2.3% to 18.2%.

### Compulsory segment of DNA Mycobacterium tuberculosis H37Rv, built in a chromosome of the recombinant strain 2-9XL Acinetobacterjohnsonii.

DEFINITION Mycobacterium tuberculosis H37Rv complete genome;
segment 111/162. ACCESSION Z83863 AL123456
VERSION Z83863.1 Gl: 3261685
KEYWORDS. SOURCE Mycobacterium tuberculosis

38881 gcgggcacca gatcgacggt gtcgcggccg aggaattcgc caccgcgctg cgccaggacg
38941 gcatgctggc gctggctcgg ttgcagcgca agatgcgctt ggtcgaatct ccctatcgca
39001 caccgcaaac gctagtcggc gggccgcgca gtgacgccgc attggctgcc tcctccggac
39061 gcacggcgac cacgaaggca atgggcaagg gctccaccca gcaccagcac ttggtgcaaa
39121 ctgaggtgcc gcgcaagctg ctagaggaat ggctggccat gtggagcggt cactaccagc
39181 tcaaagacaa actgcgcgta cagcttcggc cgcagcgggc cggctcggag gtgctcgagc
39241 tcggcatcca cggcgaaagc gatgacaagc tcgccaacgt gatattccaa ccgatccaag
39301 atcgccgcgg ccgcaccatc ctgttggtac gcgaccagaa cacgttcggt gcggaactac
39361 gccaaaagcg gctgatgacc ctgatccacc tctggctcgt ccaccgcttc aaggcgcagg
39421 cggtgcacta cgtcacgccc accgacgaca acctctacca gacctcgaag atgaagtcgc
39481 atggaatctt caccgaggtc aaccaggagg tgggcgagat catcgtcgcc gaggtgaacc
39541 acccgcgcat cgccgaactg ctgacgcccg atcgggtggc gctgcggaag ttgatcacga
39601 aggaggcgta gccagcgctg ccaactgtct tgggggccaa ccgggtgtgc gtcgaggtgg
39661 cgcacatcgc gaaacgcgaa ggatgctgtc agacggcgtc tgcggtggcc tgtcgaagat
39721 ccagcgcacc ggcgttcacc tgcgtcggcc cgcggtcgcg actaccatcg ccgcccccgt
39781 ttacggcccg gcacccggtg agaagaagcc caggagcatt tggccgatgt tgttgacgcc
39841 cgagttaaac gcagcggtga ggtgaccaac ggtgctcgtg ttgttgaagc ccgagacggt
39901 gttgcctagg ttcgccacgc ccgacgccag ctgcccgacg ttgtagattc ccgagactcc
39961 gccttgcagc gcgttcggca cctggttcca gaggcccgaa atgccgggcc cgacgttgcc
40021 gaagccggat gcgcttccat cgccactgtt gaagaagccc gaagacgggg tggtggtgga
40081 gtttccgaag cccggggcgc tcgtgatgtt gatcgggatg ttgatcggtc ccaagccgcc
40141 gttggcggtc aagttcaggg gggatccggg aatggtgaag ccggggatcg taaccgggct
40201 cgtgcccccg ctcaacggaa cattcaaccc aaacggatta atcgcgaaac cagggatcgt
40261 aaccgggctc gtgcccccgc tcaacggaac attcaaccca aacggattaa tcgcgaaacc
40321 agggatcgtg acagcgttgg tagcaccgct cagcggaata ttcaaaccga acggattaac
40381 actgaatccc tggatgccag actccagggt gccgccggcc agcgtgacgc ctaatacgaa
40441 tgtgctaagc gggatggggc cgatgtagcc cgtgaagata ccagcgacgt taaacggaag

40501 ttcgttgaga gtgatgttga ccggtatcct gatgttaatc gtaaggggga tgcgggaaat
40561 agggacgccg ggaacggtga tcggaccgac accacccagc gcgttcaggc tcaacggaat
40621 accaggaata gtaatatcag gcaccacaat cggaccgaca ccacccagcg cgttcaggct
40681 caacggaata ccaggaatag taatatccgg caccacaatc ggaccgacac cacccagcgc
40741 gttcaggctc aacggaatac caggaatagt aatatccggc accacaatcg gaccgacacc
40801 acccagcgcg ttcaggctca acggaatacc aggaatagta atatccggca ccacaatcgg
40861 accgacacca cccagcgcgt tcaggctcaa cggaatacca ggaatagtaa tatccggcac
40921 cacaatcgga ccgatgccac cattcacttc gacgctcagt gggatggcgg gaatgctgag
40981 tgtgtctgag tagccaatca gaccctggta atcgcccctc cacagtatgc cgttgctgta
41041 gctgcccgag atcagggcgc cggtgttaag gtcgccaatg tttccccagc cggtgttgag
41101 gtcgccgagg tttaggtacc ccgtgttggc gttgcccggg ttgaggtcgc ccgtgttggt
41161 gtcgccggcg ttgtagctgc ctgtgttgta gcttcctgcg ttgccgattc cagtgttgac
41221 gttgccggtg ttgaacaggc ccgtgttggc gttgcccacg ttacccaggc cggtgttgta
41281 gttgccggag ttgccgatgc cgacgtttcc gttgcctgag ttgaagaagc cgatgttgcc
41341 gttgccggag ttgaagaagc cgatgttgcc gctgccggag ttcagcgccc cgaatccgac
41401 ctgattgtcg ccggtgagcc cgataccaat atttccagtg cccgtgttgc cgaagccgat
41461 gttgccgtta ccgatgttcg cgaagccgta gttgttgccg ccgatgttcc caaagccaat
41521 gttgtgcagg gcctccgtca accccggacc cgtgtttgca aacccaaggt tgttgctgcc
41581 gacgtttcca aaaccgaagt tgttgcttcc gatgtttccg aaaccgaaac ttccgttgcc
41641 gatgtttccg ctaccgaagt tgtagctacc gacgtttccg ctacccacgt tgtagtcgcc
41701 gaggtttgcg ttgcccaagt tgagtgtgcc gtcgttggcg aagccgaagt tgaataacgt
41761 cccacctgcg gcgttgcgca tgaagccggc gagttggctg tcggtgttac cgacgccgga
41821 gtgaaaggcc gatgtcgcta ggcccagcgt gctggtgttg tagaggcctg agactgtgtt
41881 gccgaagttc aagattcccg atgtcagtgg cccgacgtta aggaatccgg agttgccgag
41941 attcccagca atgttccaga agccagatcc gcccgaaccg acgttcccga aacccgatgt
42001 gccgcccgta ccgctgttga agaagcccga tgacggggtg gtggtcgagt ttccgaagcc
42061 tggggtgccc gcgatttcga tcgggatgtt gatcggcccg aggctgccgg acacgtcgat
42121 gcccaacggg attgagggga tcgtgattgg cggggtagtg agggggccga tggcgccgcc
42181 cacatcaata cccaacggga ttgccggaag tgagtagcca tccgggaaca ccgtaaacgg
42241 gcctaaccct ccgcccacat caatacccaa cgggattgcc ggaagtgagt agccatccgg
42301 gaacaccgta aacgggccta accctccgcc cacatcaata cccaacggga ttgccggaag
42361 tgagtagcca tccgggaaca ccgtaaacgg gcctaaccct ccacccacat caatacccaa
42421 cggaatagcc ggcaaactat aaccacccga taagaaggtg atgggaccga tttgaccact

42481 cactgtcacg taatctggag ggaatccggg gaaaaatggc ggaatcgcgg gaatctcagg
42541 agtgcctagc tgtatcgata tgctacccgg gcctatgctg ccaacggtgg gatttacgcc
42601 gaataagccg atcgcaagcg gagacgcggg gatcgaaatc gatcccacgt taatgacctg
42661 gaacgccgat agctctaggc caatagaatt tagagtgatc ggcgggatgt tgatggggcc
42721 aacgagtgcc ccggtactgt tgatgcccag cccgatggcg ggaacagtaa taggcggaac
42781 attgatcggc cccaccaacg ctccggaact gttaatgccc aggccgattt cgggaatggt
42841 gatggacggg atggtgatgg ggccgacgga gccgaggccg ttgaggtcta ggccagcagc
42901 gggaatggtc agtgtgccgg agaagccgat caagccctgg tagtcgcctc gccagaagaa
42961 gccgttgctg tagttgccag agttgaatcc accggtgttg acgttgccgg tgtttcccac
43021 gccggtgttg aggttgccgg ggttgaagaa gcctgtgttg gagctgcccg tgttgaagtc
43081 gcccgtgttg aagctgccta gattgaagct gcccgtgttg tagttgcccg tgttgccgat
43141 gccagtgttg gcgatgccgg cgttgaagaa gcccgtgttg gcttggcccg tgttgccgat
43201 acccgtgttg tagctggtac ctgagttccc gatgccgaag tttccggtgc ccgtattgcc
43261 gatgccgatg ttgccggtgc ctgagttgaa caagccgata ttgccggtcc ccgagttcca
43321 gccgccgaac ccctgctggt tgtcgccggt gaggccgaag ccgatgtttc cgttgcccgt
43381 gttgccgaag ccgatgtttc cgttgccggt gttgcccaag ccaacgttgt tgctgccgac
43441 atttccaagg ccgaagttgt tgccgccggg atttaggctg cccaagttca aaatgccaag
43501 gttagcggcg cccatctgtc cgaagcccga gtttgccagg cctaagctaa gatttgccag
43561 cacacccttg gaactggtga tcgccgcggt gacgacggcc gccggagcgg ccgccaactg
43621 ggcgggcagg tctgtcagat tctgcggcgg cgcagtgaac ggcgtcaggg ccgacgccac
43681 cgccgatgcc ccggcatggt aggccgacat caccgacaca tcgatggccc acatctgttc
43741 gtatgcggcc tcaatcgcag cgatcgccgg agcattctgc ccgaagaagt ttgagaacac
43801 caatgacacc aggtcggagc ggttggccgc caccagcgcc ggttgcacca tcgccgcccg
43861 gacagcctcg aactcggcca cgagggccgc ggcttgggtt gccgaccgct gggcctgggc
43921 cgctgccgcg gcaagccatc ctaggtacgg cgctaccgca gcggccatcg ccgccgatga
43981 cggtccgagc cacgattcgc cgaccaggcc cgacgtcacg gagttgaaag aggccgctgc
44041 cgaggccaat tccatcgcca gttggtccca ggcgaccgcg gccgccgaca tgggttctga
44101 tcccgccccg ccgaatatga gggccgagtt gatctctggt ggcaatgttg aaaaattcat
44161 ggccccgact ttccctgggt gcaccgaatt catggcggct c//

### A computer protein model, read from the decoded segment

### 111/162 Mycobacterium tuberculosis H37Rv.Large fragment EcoR-EcoR

DGVAAEEFATALRQDGMLALARLQRKMRLVESPYRTPQTLVGGPRSDAALAASSGRTA -
TTKAMGKGSTQHQHLVQTEVPRKLLEEWLAMWSGHYQLKDKLRVQLRPQRAGSEVLEL
GIHGESDDKLANVIFQPIQDRRGRHU.VRDQNTFGAELRQKRLMTLIHLWLVHRFKA
QAVHYVTPTDDNLYQT3KMKSHGIFTEVNQEVGEIIVAEVNHPRIAELLTPDRVALRK
ITKEAM8AAAVAWDQLAMELASAAASFNSVT3GLVGESWLGPSSAAMAA
AVAPYLGWLAAAAAQAQRSATQAAALVAEFEAVRAAMVQPALVAANRSDLVSLVFSNF
FGQNAPAIAAIEAAYEQMWAIDVSVMSAYHAGASAVASALTPFTAPPQNLTDLPAQLA 10
AAPAAWTAAITSSKGVLANLSLGLANSGFGQMGAANLGILNLGSLNPGGNNFGLGNV
GSNNVGLGNTGNGNIGFGNTGNGNIGFGLTGDNQQGFGGWNSGTGNIGLFNSGTGNIG
IGNTGTGNFGIGNSGTSYNTGIGNTGQANTGFFNAGIANTGIGNTGNYNTGSFNLGSF
NTGDFNTGSSNTGFFNPGNLNTGVGNTGNVNTGGFNSGNYSNGFFWRGDYQGLIGFSG
TLTIPAAGLDLNGLGSVGPITIPSITIPEIGLGINSSGALVGPINVPPITVPAIGLGi 15
NSTGALVGPINIPPITLNSIGLELSAFQVINVGSISIPASPLAIGLFGVNPTVGSIGP
GSISIQLGTPEIPAiPPFFPGFPPOYVTVSGQIGPITFLSGGYSLPAIPLGIDVGGGL
GPF7YFPDGYSLPAIPLGIDVGGGLGPF7VFPDGYSLPAIPLGIDVGGGLGPFTVFPD
GYSLPAIPLGiDVGGAIGPLTTPPITIPSIPLGIDVSGSLGPINIPIEIAGTPGFGNS
TTTPSSGFFNSGTGGTSGFGNVGSGGSGFWNIAGNLGNSGFLNVGPLTSGILNFGNTV
SGLYNTSTLGLATSAFHSGVGNTDSQLAGFMRNAAGGTLFNFGFANDGTLNLGNANLG 20
DYNVGSGNVGSYNFGSGNIGNGSFGFGNIGSNNFGFGNVGSNNLGFANTGPGLTEALH
NiGFGNIGGNNYGFANIGNGNIGFGNTGTGNiGiGLTGDNQVGFGALNSGSGNIGFFN
SGNGNIGFFNSGNGNVGIGNSGNYNTGLGNVGNANTGLFNTGNVNTGIGNAGSYNTGS
YNAGDTNTGDLNPGNANTGYLNLGDLNTGWGNIGDLNTGAI.ISG8YSNGILWRGDYQG
LIGYSDTLSIPAiPLSVEVNGGIGPIWPDITIPGIPLSLNALGGVGPIWPOITIPG 25
IPLSLNALGGVGPIWPDITIPGIPLSLNALGGVGPIWPDITIPGIPLSLNALGGVG
PIWPDITIPGIPLSLNALGGVGPITVPGVPISRIPLTINIRIPVNITLNELPFNVAG
IFTGYIGPIPLSTFVLGVTLAGGTLESGIQGFSVNPFGLNIPLSGATNAVTIPGFAJN
PFGLNVPLSGGTSPVTIPGFAINPFGLNVPL8GGTSPVTIPGFTIPGSPLNLTANGGL
GPINIPINITSAPGFGNSTTTPSSGFFNSGDGSASGFGNVGPGISGLWNQVPNALQGG
VSGIYNVGQLASGVANLGNTVSGFNNTSTVGHLTAAFN8GVNNIGQMLI.GFFSPGAGP

### A potential segment of DNA Mycobacterium tuberculosis H37Rv, built in a chromosome of the recombinant strain 2-9XL Acinetobacter johnsonii

### DEFINITION Mycobacterium tuberculosis H37Rv complete genome; segment 85/162

**ACCESSION Z97193 AL123456**
**VERSION Z97193.1 01:3261816**
**KEYWORDS. SOURCE Mycobacterium tuberculosis.**

1801 ccgtcatcaa cgaattcgcg aatgccgacg cgctgcacca cggcgtcgtg gtagcgctgc
1861 accaactcgg attcgtcgac eatttcgccg gattcggtgt cgtaccaacc gggttgcggg
1921 tcgtcctccc agcggatcaa cccagtggtc caggccagct ccagcacgcc ggccgccgac
1981 agctcgtttt cgacctccat ctcgaaccgg gtgcgtgacg agccgtacgg gccgatttcg
2041 gcgccgccga cgatcaccac caggtcggcc gggtcgacat cgaggtcgtc ccattgcggc
2101 ggcggtgcgg gggtgaaacc ccggggcggc gacggcagcg cggcgatggc gccaggggcc
2161 tcggcgtcct cgtcgacggc cgccgctgcc gacatctgct cgcgcgcctt ggccgccagc
2221 tcggccatgt cgaggttggc ctcggccagg cccccggtca ggtcggcctt gatcggcgaa
2281 cgcgccgcag ccaccttgga ttccgcatca cacaggtcga gcagcagcgc cgccatctcg
2341 tcggtcgagt aggtggtgac cccggcctct tcgacggcgg ccacgatggc atcgttgtgg
2401 cccatcagcc cggtgccgcg ggtccagccg atgagcgcgt gcgccaggct gacccgtgcc
2461 gcccaggacg actcggcgtg ccagcggctc accacggcat ccagcgcgga cttggcttcg
2521 ccgtaggcgc cgtcgccgcc gaacatgcca cggttgggcg agccgggcag caccacgtgc
2581 agccgcgacg cgatgtcgcg ttcggcgccg atcgtcgaca ggccgccgat cagccgttgc
2641 acggcccaca gcagcacttt catctccatc tcggcgcgcg aaccggcctc cgacaggtcc
2701 ccgaccacgc gtggcgccgc gaacgggaac agcagcgtcg gggtctgcgc gtctttgatg
2761 tgaatcgact gcggcccaag gctttcggtc tgttcggtgc cgatccattc gaccagggcg
2821 tcgacgtcgg agtaggacgc catgttcgcc gcgaccagcc acagcgccgc gccgtaacgg
2881 gcgtggtcgc gatacagcgt gcggtagaac gccagccgct cctcgtcgag cttggaggtg
2941 gtcgcgatga cggtggctcc gccgtcgagc agccgagcca ccaccgacgc ggcgatcgaa
3001 cccttcgaag cgccggtcac cacggcaact tcgccgccgt agcggccggg ttcggggttc

### A computer model of the protein fragment, read from the decoded segment 85/162 Mycobacterium tuberculosis H37Rv. Small fragment EcoR-EcoR

AEFRRSLDRVMPRDADPDLIIGRYIPNLVPRLFTLD
RDFIQEIRDLVPAEPLDEILADYDTWLRERPREMARTVFIELLAWQFASPVRWIETQD
LLFIEEAAGGLGVERFVEIGVKSSPWAGLATNTLKLPEYAHSTVEVLNAERDAAVLF
ATDTDPEPEPEEDEPVAESPAPDWSEAAPVAPAASSAGPRPDDLVFDAADATLALIA
LSAKMRIDQIEELDSIESITDGASSRRNQLLVDLGSELNLGAIDGAAESDLAGLRSQV
TKLARTYKPYGPVLSDAINDQLRTVLGPSGKRPGAIAERVKKTWELGEGWAKHVTVEV
ALGTREGSSVRGGAMGHLHEGALADAASVDKVIDAAVASVAARQGVSVALPSAGSGGG
ATIDAAALSEFTDQITGREGVLASAARLVLGQLGLDDPVNALPAAPDSELIDLVTAEL
GADWPRLVAPVFDPKKAWFDDRWASAREDLVKLWLTDEGDIDADWPRLAERFEGAGH
WATQATWWQGKSLAAGRQIHASLYGRIAAGAENPEPGRYGGEVAWTGASKGSIAAS
WARLLDGGATVIATTSKLDEERLAFYRTLYRDHARYGAALWLVAANMASYSDVDALV
EWIGTEQTESLGPQSIHIKDAQTPTLLFPFAAPRWGDLSEAGSRAEM EM KVLLWAVQ
RLIGGLSTIGAERDIASRLHWLPGSPNRGMFGGDGAYGEAKSALDAWSRWHAESSW
AARVSLAHALIGWTRGTGLMGHNDAIVAAVEEAGVTTYSTDEMAALLLDLCDAESKVA
AARSPIKADLTGGLAEANLDMAELAAKAREQMSAAAAVDEDAEAPGAIAALPSPPRGF
TPAPPPQWDDLDVDPADLW1VGGAEIGPYGSSRTRFEMEVENELSAAGVLELAWTTG
LIRWEDDPQPGWYDTESGEMVDESELVQRYHDAWQRVGIREFVDDGAIDPDHASPLL

Перечень последовательностей

<110> Общество с ограниченной ответственностью
«БИОТЭК»; Кисличкин Николай Николаевич
Obschestvo s ogranichennoy otvetstvennostyu «BIOTEK»;
Kislichkin Nikolay Nikolaevich

<120> Средство, обладающее свойством формировать клеточный
иммунитет против Mycobacterium tuberculosis H37 Rv, способ
получения его (варианты), рекомбинантный штамм и средство
для диагностики туберкулеза.

<130>

<150> RU2007117342

<151> 2007-05-10

<160> 2

<210> 1

<211> 5321

<212> DNA

<213> Mycobacterium tuberculosis H37Rv

<400> 1

gcgggcacca gatcgacggt gtcgcggccg aggaattcgc caccgcgctg cgccaggacg 60
gcatgctggc gctggctcgg ttgcagcgca agatgcgctt ggtcgaatct ccctatcgca 120
caccgcaaac gctagtcggc gggccgcgca gtgacgccgc attggctgcc tcctccggac 180
gcacggcgac cacgaaggca atgggcaagg gctccaccca gcaccagcac ttggtgcaaa 240
ctgaggtgcc gcgcaagctg ctagaggaat ggctggccat gtggagcggt cactaccagc 300
tcaaagacaa actgcgcgta cagcttcggc cgcagcgggc cggctcggag gtgctcgagc 360
tcggcatcca cggcgaaagc gatgacaagc tcgccaacgt gatattccaa ccgatccaag 420
atcgccgcgg ccgcaccatc ctgttggtac gcgaccagaa cacgttcggt gcggaactac 480
gccaaaagcg gctgatgacc ctgatccacc tctggctcgt ccaccgcttc aaggcgcagg 540
cggtgcacta cgtcacgccc accgacgaca acctctacca gacctcgaag atgaagtcgc 600
atggaatctt caccgaggtc aaccaggagg tgggcgagat catcgtcgcc gaggtgaacc 660
acccgcgcat cgccgaactg ctgacgcccg atcgggtggc gctgcggaag ttgatcacga 720

aggaggcgta gccagcgctg ccaactgtct tgggggccaa ccgggtgtgc gtcgaggtgg 780
cgcacatcgc gaaacgcgaa ggatgctgtc agacggcgtc tgcggtggcc tgtcgaagat 840
ccagcgcacc ggcgttcacc tgcgtcggcc cgcggtcgcg actaccatcg ccgcccccgt 900
ttacggcccg gcacccggtg agaagaagcc caggagcatt tggccgatgt tgttgacgcc 960
cgagttaaac gcagcggtga ggtgaccaac ggtgctcgtg ttgttgaagc ccgagacggt 1020
gttgcctagg ttcgccacgc ccgacgccag ctgcccgacg ttgtagattc ccgagactcc 1080
gccttgcagc gcgttcggca cctggttcca gaggcccgaa atgccgggcc cgacgttgcc 1140
gaagccggat gcgcttccat cgccactgtt gaagaagccc gaagacgggg tggtggtgga 1200
gtttccgaag cccggggcgc tcgtgatgtt gatcgggatg ttgatcggtc ccaagccgcc 1260
gttggcggtc aagttcaggg gggatccggg aatggtgaag ccggggatcg taaccgggct 1320
cgtgcccccg ctcaacggaa cattcaaccc aaacggatta atcgcgaaac cagggatcgt 1380
aaccgggctc gtgcccccgc tcaacggaac attcaaccca aacggattaa tcgcgaaacc 1440
agggatcgtg acagcgttgg tagcaccgct cagcggaata ttcaaaccga acggattaac 1500
actgaatccc tggatgccag actccagggt gccgccggcc agcgtgacgc ctaatacgaa 1560
tgtgctaagc gggatggggc cgatgtagcc cgtgaagata ccagcgacgt taaacggaag 1620
ttcgttgaga gtgatgttga ccggtatcct gatgttaatc gtaaggggga tgcgggaaat 1680
agggacgccg ggaacggtga tcggaccgac accacccagc gcgttcaggc tcaacggaat 1740
accaggaata gtaatatcag gcaccacaat cggaccgaca ccacccagcg cgttcaggct 1800
caacggaata ccaggaatag taatatccgg caccacaatc ggaccgacac cacccagcgc 1860
gttcaggctc aacggaatac caggaatagt aatatccggc accacaatcg gaccgacacc 1920
acccagcgcg ttcaggctca acggaatacc aggaatagta atatccggca ccacaatcgg 1980
accgacacca cccagcgcgt tcaggctcaa cggaatacca ggaatagtaa tatccggcac 2040
cacaatcgga ccgatgccac cattcacttc gacgctcagt gggatggcgg gaatgctgag 2100
tgtgtctgag tagccaatca gaccctggta atcgcccctc cacagtatgc cgttgctgta 2160
gctgcccgag atcagggcgc cggtgttaag gtcgccaatg tttccccagc cggtgttgag 2220
gtcgccgagg tttaggtacc ccgtgttggc gttgcccggg ttgaggtcgc ccgtgttggt 2280
gtcgccggcg ttgtagctgc ctgtgttgtagcttcctgcg ttgccgattc cagtgttgac 2340
gttgccggtg ttgaacaggc ccgtgttggc gttgcccacg ttacccaggc cggtgttgta 2400
gttgccggag ttgccgatgc cgacgtttcc gttgcctgag ttgaagaagc cgatgttgcc 2460
gttgccggag ttgaagaagc cgatgttgcc gctgccggag ttcagcgccc cgaatccgac 2520
ctgattgtcg ccggtgagcc cgataccaat atttccagtg cccgtgttgc cgaagccgat 2580
gttgccgtta ccgatgttcg cgaagccgta gttgttgccg ccgatgttcc caaagccaat 2640
gttgtgcagg gcctccgtca accccggacc cgt gtttgca aacccaaggt tgttgctgcc 2700
gacgtttcca aaaccgaagt tgttgcttcc gatgtttccg aaaccgaaac ttccgttgcc 2760

gatgtttccg ctaccgaagt tgtagctacc gacgtttccg ctacccacgt tgtagtcgcc 2820
gaggtttgcg ttgcccaagt tgagtgtgcc gtcgttggcg aagccgaagt tgaataacgt 2880
cccacctgcg gcgttgcgca tgaagccggc gagttggctg tcggtgttac cgacgccgga 2940
gtgaaaggcc gatgtcgcta ggcccagcgt gctggtgttg tagaggcctg agactgtgtt 3000
gccgaagttc aagattcccg atgtcagtgg cccgacgtta aggaatccgg agttgccgag 3060
attcccagca atgttccaga agccagatcc gcccgaaccg acgttcccga aacccgatgt 3120
gccgcccgta ccgctgttga agaagcccga tgacggggtg gtggtcgagt ttccgaagcc 3180
tggggtgccc gcgatttcga tcgggatgtt gatcggcccg aggctgccgg acacgtcgat 3240
gcccaacggg attgagggga tcgtgattgg cggggtagtg agggggccga tggcgccgcc 3300
cacatcaata cccaacggga ttgccggaag tgagtagcca tccgggaaca ccgtaaacgg 3360
gcctaaccct ccgcccacat caatacccaa cgggattgcc ggaagtgagt agccatccgg 3420
gaacaccgta aacgggccta accctccgcc cacatcaata cccaacggga ttgccggaag 3480
tgagtagcca tccgggaaca ccgtaaacgg gcctaaccct ccacccacat caatacccaa 3540
cggaatagcc ggcaaactat aaccacccga taagaaggtg atgggaccga tttgaccact 3600
cactgtcacg taatctggag ggaatccggg ga aaaatggc ggaatcgcgg gaatctcagg 3660
agtgcctagc tgtatcgata tgctacccgg gcctatgctg ccaacggtgg gatttacgcc 3720
gaataagccg atcgcaagcg gagacgcggg gatcgaaatc gatcccacgt taatgacctg 3780
gaacgccgat agctctaggc caatagaatt tagagtgatc ggcgggatgt tgatggggcc 3840
aacgagtgcc ccggtactgt tgatgcccag cccgatggcg ggaacagtaa taggcggaac 3900
attgatcggc cccaccaacg ctccggaact gttaatgccc aggccgattt cgggaatggt 3960
gatggacggg atggtgatgg ggccgacgga gccgaggccg ttgaggtcta ggccagcagc 4020
gggaatggtc agtgtgccgg agaagccgat caagccctgg tagtcgcctc gccagaagaa 4080
gccgttgctg tagttgccag agttgaatcc accggtgttg acgttgccgg tgtttcccac 4140
gccggtgttg aggttgccgg ggttgaagaa gcctgtgttg gagctgcccg tgttgaagtc 4200
gcccgtgttg aagctgccta gattgaagct gcccgtgttg tagttgcccg tgttgccgat 4260
gccagtgttg gcgatgccgg cgttgaagaa gcccgtgttg gcttggcccg tgttgccgat 4320
acccgtgttg tagctggtac ctgagttccc gatgccgaag tttccggtgc ccgtattgcc 4380
gatgccgatg ttgccggtgc ctgagttgaa caagccgata ttgccggtcc ccgagttcca 4440
gccgccgaac ccctgctggt tgtcgccggt gaggccgaag ccgatgtttc cgttgcccgt 4500
gttgccgaag ccgatgtttc cgttgccggt gttgcccaag ccaacgttgt tgctgccgac 4560
atttccaagg ccgaagttgt tgccgccggg atttaggctg cccaagttca aaatgccaag 4620
gttagcggcg cccatctgtc cgaagcccga gtttgccagg cctaagctaa gatttgccag 4680
cacacccttg gaactggtga tcgccgcggt gacgacggcc gccggagcgg ccgccaactg 4740
ggcgggcagg tctgtcagat tctgcggcgg cgcagtgaac ggcgtcaggg ccgacgccac 4800

cgccgatgcc ccggcatggt aggccgacat caccgacaca tcgatggccc acatctgttc 4860
gtatgcggcc tcaatcgcag cgatcgccgg agcattctgc ccgaagaagt ttgagaacac 4920
caatgacacc aggtcggagc ggttggccgc caccagcgcc ggttgcacca tcgccgcccg 4980
gacagcctcg aactcggcca cgagggccgc ggcttgggtt gccgaccgct gggcctgggc 5040
cgctgccgcg gcaagccatc ctaggtacgg cgctaccgca gcggccatcg ccgccgatga 5100
cggtccgagc cacgattcgc cgaccaggcc cgacgtcacg gagttgaaag aggccgctgc 5160
cgaggccaat tccatcgcca gttggtccca ggcgaccgcg gccgccgaca tgggttctga 5220
tcccgccccg ccgaatatga gggccgagtt gatctctggt ggcaatgttg aaaaattcat 5280
ggccccgact ttccctgggt gcaccgaatt catggcggct c 5321

<130>

<150> RU2007117342

<151> 2007-05-10

<210> 2

<211> 1280

<212> DNA

<213> Mycobacterium tuberculosis H37Rv

<400> 2
ccgtcatcaa cgaattcgcg aatgccgacg cgctgcacca cggcgtcgtg gtagcgctgc 60
accaactcgg attcgtcgac catttcgccg gattcggtgt cgtaccaacc gggttgcggg 120
tcgtcctccc agcggatcaa cccagtggtc caggccagct ccagcacgcc ggccgccgac 180
agctcgtttt cgacctccat ctcgaaccgg gtgcgtgacg agccgtacgg gccgatttcg 240
gcgccgccga cgatcaccac caggtcggcc gggtcgacat cgaggtcgtc ccattgcggc 300
ggcggtgcgg gggtgaaacc ccggggcggc gacggcagcg cggcgatggc gccaggggcc 360
tcggcgtcct cgtcgacggc cgccgctgcc gacatctgct cgcgcgcctt ggccgccagc 420
tcggccatgt cgaggttggc ctcggccagg cccccggtca ggtcggcctt gatcggcgaa 480
cgcgccgcag ccaccttgga ttccgcatca cacaggtcga gcagcagcgc cgccatctcg 540
tcggtcgagt aggtggtgac cccggcctct tcgacggcgg ccacgatggc atcgttgtgg 600
cccatcagcc cggtgccgcg ggtccagccg atgagcgcgt gcgccaggct gacccgtgcc 660
gcccaggacg actcggcgtg ccagcggctc accacggcat ccagcgcgga cttggcttcg 720
ccgtaggcgc cgtcgccgcc gaacatgcca cggttgggcg agccgggcag caccacgtgc 780
agccgcgacg cgatgtcgcg ttcggcgccg atcgtcgaca ggccgccgat cagccgttgc 840

acggcccaca gcagcacttt catctccatc tcggcgcgcg aaccggcctc cgacaggtcc 900
ccgaccacgc gtggcgccgc gaacgggaac agcagcgtcg gggtctgcgc gtctttgatg 960
tgaatcgact gcggcccaag gctttcggtc tgttcggtgc cgatccattc gaccagggcg 1020
tcgacgtcgg agtaggacgc catgttcgcc gcgaccagcc acagcgccgc gccgtaacgg 1080
gcgtggtcgc gatacagcgt gcggtagaac gccagccgct cctcgtcgag cttggaggtg 1140
gtcgcgatga cggtggctcc gccgtcgagc agccgagcca ccaccgacgc ggcgatcgaa 1200
cccttcgaag cgccggtcac cacggcaact tcgccgccgt agcggccggg ttcggggttc 1260

## Claims

1. The recombinant strain 2-9XL Acinetobacter johnsonii VKPM-9312 - producer of type- specific glycoprotein tuberculosis complex Mycobacterium tuberculosis H37 Rv (TB-antigen), deposited in the Research Institute for Genetics and Selection of Industrial Microorganisms.

2. Method of producing a type-specific glycoprotein tuberculosis complex Mycobacterium tuberculosis H37 Rv (TB- antigen), **characterizing by** that a culture of the recombinant strain 2-9XL Acinetobacter johnsonii is fermented on the growth medium at 32°C within 2-3 days, washed off by a physiological solution, the produced suspension of bacteria is centrifuged, the residue is suspended in water at 4°C, processed by NaOH solution to pH 6.0-9.0, in 15-16 hours the produced suspension is centrifuged, the residue is repeatedly processed by NaOH solution, and a supernatant fluid, containing a dissolved bacteria capsule of the strain 2-9XL, neutralized by 0,5 N solution TCA to pH 6.0, centrifuged, and to the supernatant fluid added is 0.5 N solution TCA to pH 3.0-3.5, the solution is sustained 15-15 hours to produce a complex of TB -antigen, at that with a portion of the residual, repeatedly processed by NaOH solution, neutralized are by TCA solution with the stated sequence the 1^{st} and 2^{nd} solutions with TB-antigen complex, precipitated after TCA processing, centrifuged and the produced residues containing TB-antigen, are dissolved in PBS-buffer, solutions are combined and purified by fractioning a gel-filtration on Sephadex G-200 or by means of an ion-change chromatography, eluates contain the refined TB - antigen with a molecular mass from 55.0 to 75.0 KDA and available carbohydrates from 20 to 50%.

3. The method of producing a type-specific glycoprotein tuberculosis complex Mycobacterium tuberculosis H37 Rv (TB-antigen), **characterizing by** that a culture of the recombinant strain 2-9XL Acinetobacter johnsonii is fermented on the growth medium at 32°C within 3 days, washed off by a physiological solution, the produced bacteria suspension is centrifuged, the residue is suspended in water at 4°C, processed by NaOH solution to pH 6.0-9.0, in 15-16 hours the produced suspension is centrifuged, the residue is repeatedly processed by NaOH solution, and a supernatant liquid, containing a dissolved bacteria capsule of the strain 2-9XL, is neutralized by 0.5 N solution TCA to pH 6.0, added is ammonium sulfate to 30% saturation, sustained at 4°C, centrifuged, to the supernatant liquid added is ammonium sulfate to 100% saturation, sustained at 4°C, at that with a residual portion, repeatedly processed by NaOH solution, is neutralized by means of TCA and a precipitation of ammonium sulfate in the above-stated sequence, two residues, produced after 100% saturation of ammonium sulfate, is dissolved in PBS-buffer pH 7.0, is dialyzed against PBS buffer great volume, concentrated solutions, containing TB-antigen, are combined and purified by the gel-filtration fractioning on Sephadex G-200 or by means of an ion-change chromatography, eluates contain the refined TB-antigen with a molecular mass from 55.0 to 75.0 KDA and available carbohydrates from 20 to 50%.

4. The tool to form B- and T- cellular immunity against Mycobacterium tuberculosis H37 Rv, containing type-specific glycoprotein tuberculosis complex (TB-antigen) Mycobacterium tuberculosis H37 Rv, produced by the strain 2-9XL Acinetobacter johnsonii, **characterized in** pt.1.

5. The tool to diagnose tuberculosis, containing type-specific glycoprotein tuberculosis complex (TB-antigen) Mycobacterium tuberculosis H37 Rv, produced by the method under pt. 2.
